# FASCICULE DE BREVET EUROPEEN

(11) **EP 0 401 139 B1**
(45) Date de publication et mention de la délivrance du brevet: **17.02.1993**
(21) Numéro de dépôt: 90420263.7
(22) Date de dépôt: 01.06.1990
(51) Int. Cl.: A61M 1/16

(54) **Dispositif et procédé de mesure de l'ultrafiltration dans un rein artificiel**
Gerät und Verfahren für die Ultrafilterungsmessung in einer Kunstniere
Apparatus and method for the measurement of ultrafiltrate in an artificial kidney

(30) Priorité: 02.06.1989 FR 8907567
(43) Date de publication de la demande: 05.12.1990
(73) Titulaire: HOSPAL INDUSTRIE, F-69883 Meyzieu Cédex (FR)
(72) Inventeur: Chevallet, Jacques, F-63360 Serezin du Rhône (FR)

(56) Documents cités:
- EP-A- 0 213 050

## Description

La présente invention concerne le domaine technique du traitement extracorporel du sang par un rein artificiel. Plus particulièrement, la présente invention concerne un dispositif et un procédé de mesure de la quantité d'ultrafiltration.

La demande de brevet EP 213.050 décrit un dispositif permettant de mesurer l'ultrafiltration du sang en mesurant la quantité de liquide extraite du circuit de liquide de dialyse au moyen d'une pompe d'ultrafiltration. Le dispositif est constitué essentiellement de deux récipients possédant chacun une sonde de mesure capables d'indiquer l'atteinte d'un niveau prédéterminé dans chacun des récipients. Chaque récipient est rempli grâce au liquide pompé par la pompe d'ultrafiltration. Un jeu de vannes contrôlées de façon appropriée par un organe de contrôle permet d'effectuer le remplissage d'un récipient pendant la vidange de l'autre récipient et vice-versa. Les sondes envoient à l'organe un signal électrique à chaque fois que le niveau prédéterminé est atteint ; cet organe, connaissant le volume de chaque récipient, peut alors effectuer le calcul de la quantité de liquide ultrafiltré. Un tel dispositif manque cependant de précision dans le cas où le liquide pompé contient des bulles qui perturbent la détection du niveau par les sondes de mesure. En outre, une vitesse élevée de remplissage des récipients est un facteur critique qui rend la détection difficile et, dans le cas où le liquide mesuré provient d'une pompe d'ultrafiltration à pulsations, la détection du niveau du liquide dans les récipients est perturbée. L'objet de la présente invention est donc de pallier aux inconvénients de l'art antérieur et de proposer un dispositif et un procédé de mesure de l'ultrafiltration permettant de s'affranchir de la présence de bulles dans le liquide extrait du circuit de liquide de dialyse.

Un autre objet de la présente invention est de proposer un dispositif et un procédé de mesure de l'ultrafiltration peu sensible aux pulsations du liquide mesuré.

Un autre objet de la présente invention est de proposer un dispositif et un procédé de mesure de l'ultrafiltration permettant de réduire la vitesse de remplissage des récipients à l'approche des dispositifs de détection de niveau.

Afin d'atteindre ces objets, la présente invention propose un dispositif de mesure, dans un rein artificiel, de la quantité de liquide extraite du circuit de liquide de dialyse et correspondant à l'ultrafiltration du sang, comprenant :
- au moins un récipient de mesure destiné à recevoir le liquide extrait, ledit récipient étant pourvu d'au moins un détecteur de niveau haut capable de détecter la présence de liquide à un niveau prédéterminé
- des moyens de vidange dudit récipient de mesure, caractérisé en ce qu'il comporte en outre :
- un récipient tampon relié d'une part au circuit de liquide de dialyse et relié d'autre part au récipient de mesure afin d'assurer son remplissage, ce récipient tampon étant positionné de façon à permettre le remplissage par gravité du récipient de mesure .

La présente invention a également pour objet un procédé de mesure, dans un rein artificiel, de la quantité de liquide extraite du circuit de liquide de dialyse et correspondant à l'ultrafiltration du sang consistant:
- à remplir au moins un récipient de mesure à partir du liquide à mesurer,
- à détecter la présence de liquide à un niveau haut prédéterminé dans ledit récipient,
- à vider le récipient de mesure (1,2),
   caractérisé en ce qu'il consiste en outre à réduire la vitesse de remplissage dudit récipient de mesure à l'approche dudit niveau prédéterminé.

D'autres objets et avantages apparaîtront plus clairement à la lecture de la description qui va suivre en référence aux figures jointes qui illustrent schématiquement, à titre d'exemples, et sans échelle déterminée, deux modes de réalisation de la présente invention.

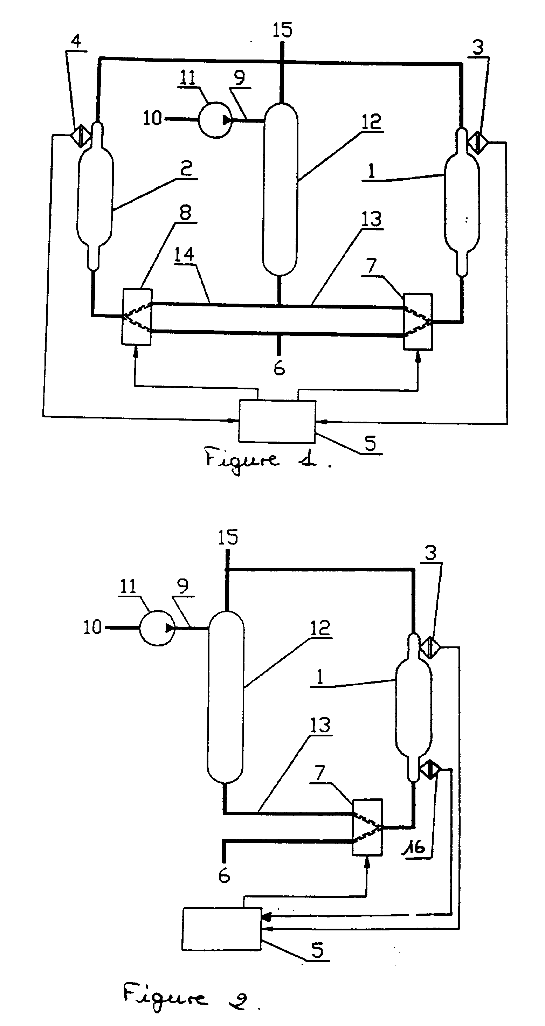

Le dispositif de mesure représenté à la figure 1 comprend, de façon connue en soi, un premier récipient de mesure 1 et un second récipient de mesure 2 constitués par exemple par des réservoirs verticaux cylindriques. Les récipients sont munis chacun à leur extrémité supérieure de tubes verticaux de section droite moindre, autour desquels sont disposés des détecteurs de niveau 3 et 4 reliés par des connections appropriées à un organe de contrôle 5.

L'extrémité inférieure du récipient 1 (respectivement du récipient 2) est mise en communication sélectivement soit avec une source du liquide à mesurer, soit avec des moyens d'évacuation 6 conduisant par exemple à l'égoût, grâce à une vanne 3 voies 7 (respectivement 8). Les vannes 7 et 8 sont avantageusement des électrovannes pilotées par l'organe de contrôle 5. Le liquide dont on veut mesurer le volume s'écoule dans une canalisation 9 en provenance du circuit de liquide de dialyse d'un rein artificiel 10 (non représenté) ; ce liquide peut être mis en circulation par tous moyens appropriés et par exemple, grâce à une pompe d'ultrafiltration 11.

Selon l'invention, le dispositif de mesure de la quantité de liquide s'écoulant dans la canalisation 9 comporte en outre un récipient tampon 12 qui peut être du même type que les récipients 1 et 2. Ce récipient tampon 12 est relié au circuit 10 via la pompe 11 grâce à la canalisation 9 qui débouche préférentiellement dans sa partie supérieure.

La partie inférieure du récipient 12 est reliée d'une part par une canalisation 13 à la vanne 7 susceptible de mettre en communication le récipient 12 avec le récipient 1, et d'autre part, par une canalisation 14 à la vanne 8 susceptible de mettre en communication le récipient 12 avec le récipient 2. De façon avantageuse, les trois récipients 1, 2 et 12 sont reliés par un dispositif approprié 15 (non représenté) à la pression atmosphérique.

Le fonctionnement du dispositif de mesure selon l'invention est le suivant. Le liquide provenant du circuit de liquide de dialyse et correspondant à l'ultrafiltration du sang est mis en circulation grâce, par exemple, à la pompe 11 et s'écoule tout d'abord dans le récipient tampon 12. L'ouverture du récipient 12 à l'atmosphère permet d'assurer le dégazage du liquide extrait du circuit de liquide de dialyse. Ce dégazage est encore favorisé par le fait que le récipient 12 est alimenté en liquide à mesurer par sa partie supérieure. Ainsi, dans le cas où le liquide extrait par la pompe 11 contient des bulles, le dégazage effectué permet d'éviter que ces bulles ne perturbent la détection de niveau par les détecteurs 3 et 4. Dans le cas où la position de la vanne 7 est telle que la canalisation 13 est mise en communication avec le récipient 1, le liquide à mesurer après un passage dans le récipient tampon 12, va remplir le récipient de mesure 1. Pendant cette opération de remplissage du récipient de mesure 1, la position de la vanne 8 est telle que la communication entre le récipient tampon 12 et le récipient 2 est coupée, alors que le récipient 2 est mis en communication avec les moyens d'évacuation 6 tels que l'égoût. Le remplissage du récipient 1 s'effectue parallèlement à la montée du liquide dans le récipient 12, à une vitesse lente, fonction notamment du débit d'ultrafiltration, c'est-à-dire de l'extraction du liquide dans la canalisation 9. Lorsque le liquide atteint le niveau du détecteur 3, celui-ci transmet un signal électrique à l'organe de contrôle 5 qui peut ainsi, connaissant la capacité du récipient ainsi que des différentes canalisations remplies de liquide, calculer la valeur de l'ultrafiltration effectuée et éventuellement provoquer son affichage. Le signal électrique transmis par le détecteur 3 à l'organe 5 provoque également de la part de ce dernier une action sur les vannes 7 et 8, de façon à mettre le récipient de mesure 1 en communication non plus avec le récipient tampon 12 mais avec les moyens d'évacuation 6 et le récipient de mesure 2 en communication non plus avec les moyens d'évacuation 6 mais avec le récipient tampon 12. Le récipient 1 va ainsi se trouver en phase de vidange et le récipient 2 en phase de remplissage. Le remplissage du récipient 2 est effectué au début grâce au liquide déjà présent dans le réservoir tampon 12 qui s'écoule par gravité, selon le principe des vases communicants à une vitesse assez rapide. Par contre, la fin du remplissage s'effectue à une vitesse lente qui dépend notamment du débit d'ultrafiltration. En effet, la montée du liquide dans le récipient 2 a lieu parallèlement avec la montée du liquide dans le récipient tampon 12. Ainsi, si l'on choisit un récipient tampon 12 avec une section large, du moins au niveau des détecteurs 3 et 4, la vitesse finale de remplissage sera lente, malgré le fait que le liquide circule dans une canalisation étroite autour de laquelle est placé le détecteur de niveau 4. La section étroite de la canalisation dans laquelle circule le liquide au moment de sa détection permet une bonne précision du volume mesuré alors que la vitesse lente permet une bonne détection du niveau. Lorsque le liquide atteint le niveau du détecteur 4, celui-ci émet un signal électrique qui est transmis à l'organe 5 qui va à nouveau inverser les ouvertures et fermetures des vannes 7 et 8, de façon à provoquer la vidange du récipient 2 et le remplissage du récipient 1. En outre, l'organe 5, connaissant la capacité du récipient 2, va incrémenter la valeur de l'ultrafiltration mesurée du volume du récipient 2. Cette nouvelle valeur de l'ultrafiltration peut alors être affichée. Le fonctionnement du dispositif de mesure selon l'invention se poursuit ainsi par remplissage et vidange successifs alternativement d'un récipient de mesure puis de l'autre, l'un des récipients étant en phase de vidange lorsque l'autre est en phase de remplissage et vice-versa.

La montée lente du liquide en fin de phase de remplissage permet très avantageusement une très bonne détection de l'atteinte du niveau prédéterminé dans l'un ou l'autre des récipients. En outre, l'ouverture à l'atmosphère du récipient tampon 12 et son alimentation en liquide par sa partie supérieure permettent d'effectuer un très bon dégazage du liquide à mesurer, ce qui évite les erreurs de mesure provenant d'une mauvaise détection de l'atteinte du niveau prédéterminé dans chacun des récipients de mesure, à cause de la présence de bulles. Le récipient tampon 12 constitue en outre une sorte de réservoir permettant d'atténuer les perturbations pouvant provenir des pulsations éventuelles de la pompe d'ultrafiltration 11.

Des essais réalisés avec un dispositif de mesure selon la présente invention ont permis d'effectuer des mesures avec une précision de 1 pour mille.

Le dispositif de mesure de la présente invention a été décrit en relation avec des vannes 3 voies 7 et 8, qui peuvent naturellement être remplacées chacune par 2 vannes 2 voies.

Les capacités respectives des deux récipients de mesure 1 et 2 ne sont pas un facteur critique pour la présente invention. Cependant, étant donné que l'information concernant l'ultrafiltration réalisée est donnée à chaque nouveau remplissage d'un récipient de mesure, il est souhaitable de donner des informations le plus fréquemment possible et donc d'avoir des récipients de faible capacité. Mais d'autre part, chaque commutation des vannes 7 et 8 entraîne une erreur, même faible, de la mesure effectuée. Afin de réduire cette erreur autant que possible, il est donc souhaitable de réduire le nombre de commutations et de prévoir par conséquent des récipients de grande capacité. Le choix de la capacité des récipients peut donc être considéré comme un compromis entre ces deux exigences, et on a remarqué qu'une capacité de l'ordre de 20 ml est satisfaisante. Ces deux récipients peuvent être ou non de capacité égale. Leur étalonnage peut être effectué par tout moyen connu ; il est possible d'étalonner chacun d'entre eux, ou simplement l'ensemble des deux. Dans ce dernier cas, si les deux récipients sont approximativement de même capacité, la valeur de l'étalonnage peut être divisée par 2 pour fournir à l'organe de contrôle 5 la valeur de remplissage de chacun des récipients, ce qui permet à l'ultrafiltration d'être incrémentée à chaque nouveau remplissage de l'un ou l'autre des récipients. Il est également possible de n'incrémenter la valeur de l'ultrafiltration qu'à chaque nouveau remplissage des deux récipients ; par exemple chaque fois que le récipient 1 est rempli, la valeur de l'ultrafiltration est incrémentée par l'organe 5 de la valeur de la capacité des deux récipients.

La forme du récipient tampon 12 peut également faire l'objet de nombreuses variantes de réalisation, la caractéristique avantageuse de ce récipient 12 étant toutefois d'avoir une section droite importante au niveau des détecteurs 3 et 4 afin de réduire la vitesse de fin de remplissage des récipients de mesure 1 et 2.

L'alimentation en liquide à mesurer a été décrite par la partie supérieure du récipient 12, ce qui est une solution particulièrement avantageuse pour assurer un bon dégazage. Il est cependant possible d'effectuer cette alimentation à n'importe quel niveau du récipient tampon 12, et d'assurer le dégazage du liquide à mesurer grâce à un dispositif particulier en amont du dispositif de mesure.

Le dispositif de mesure de la présente invention a été décrit en relation avec deux récipients de mesure (1,2) pour recevoir le liquide extrait du circuit de liquide de dialyse.
Cependant, ce dispositif peut fonctionner avec un nombre plus élevé de récipients, se remplissant et se vidangeant successivement. Il peut également être utilisé dans certains cas, notamment dans les cas où le débit d'ultrafiltration est faible, avec un seul récipient de mesure du liquide extrait. Ce cas est illustré à la figure 2 où les éléments identiques à ceux représentés sur la figure 1 sont identifiés par les mêmes numéros de référence. Dans ce cas, le récipient tampon 12 doit présenter une capacité suffisante pour recevoir le liquide durant la phase de vidange du récipient de mesure 1.
En effet, le fonctionnement du dispositif est le suivant : le liquide extrait par la pompe 11 du circuit de liquide de dialyse et correspondant à l'ultrafiltration du sang est reçu dans le récipient tampon 12 où il est dégazé. Le liquide est ensuite transmis au récipient de mesure 1 qui se remplit jusqu'au moment où le liquide atteind le niveau du détecteur 3. A ce moment, l'organe de contrôle 5 fait basculer la vanne 7 de manière à obturer la communication 13 entre le récipient tampon 12 et le récipient 1, et à provoquer la vidange du récipient de mesure 1.
Durant ce temps de vidange, le liquide extrait par la pompe 11 s'accumule dans le récipient tampon 12 puis lorsque le récipient de mesure 1 est vidé, l'organe de contrôle 5 agit sur la vanne 7 afin d'interrompre la communication entre le récipient de mesure 1 et les moyens d'évacuation 6, et de mettre en communication le récipient tampon 12 et le récipient 1. Le récipient 1 se remplit alors à partir du récipient 12, tout d'abord rapidement, puis plus lentement à l'approche du détecteur de niveau 3. L'information concernant la vidange du récipient de mesure 1 peut être transmise à l'organe de contrôle 5 grâce à un détecteur de niveau bas 16.

De nombreuses variantes de réalisation sont ainsi à la portée du technicien sans pour autant sortir du cadre de la présente invention telle que définie par les revendications.

## Revendications

1. Dispositif de mesure, dans un rein artificiel, de la quantité de liquide extraite du circuit de liquide de dialyse (10) et correspondant à l'ultrafiltration du sang, comprenant :
- au moins un récipient de mesure (1,2) destiné à recevoir le liquide extrait, ledit récipient (1,2) étant pourvu d'au moins un détecteur de niveau haut (3,4) capable de détecter la présence de liquide à un niveau prédéterminé,
- des moyens de vidange (6,7,8) dudit récipient de mesure,
caractérisé en ce qu'il comporte en outre :
- un récipient tampon (12) relié d'une part au circuit de liquide de dialyse (10) et relié d'autre part au récipient de mesure (1,2) afin d'assurer son remplissage, ce récipient tampon étant positionné de façon à permettre le remplissage par gravité du récipient de mesure (1,2).

2. Dispositif selon la revendication 1, caractérisé en ce qu'il comporte un premier (1) et un second (2) récipients de mesure qui sont alternativement remplis à partir du récipient tampon (12).

3. Dispositif selon l'une des revendications 1 et 2, caractérisé en ce que lesdits récipients (1,2,12) sont reliés à la pression atmosphérique.

4. Dispositif selon l'une des revendications précédentes, caractérisé en ce que les récipients de mesure (1,2) sont munis d'au moins une partie dont la section droite est inférieure à la section droite moyenne desdits récipients, lesdits détecteurs de niveau (3,4,16) étant situés sur cette partie de section droite inférieure.

5. Dispositif selon l'une des revendications précédentes, caractérisé en ce que le récipient tampon (12) possède au moins une portion située au niveau desdits détecteurs (3,4,16) dont la section droite est supérieure ou égale à la section droite des récipients de mesure (1,2) audit niveau.

6. Dispositif selon l'une des revendications précédentes, caractérisé en ce que ledit récipient tampon (12) est relié à sa partie supérieure audit circuit de liquide de dialyse (10).

7. Dispositif selon l'une des revendications précédentes, caractérisé en ce que lesdits récipients (1, 2, 12) sont mis en communication par leur partie inférieure.

8. Procédé de mesure, dans un rein artificiel, de la quantité de liquide extraite du circuit de liquide de dialyse (10) et correspondant à l'ultrafiltration du sang consistant:
- à remplir au moins un récipient de mesure (1,2) à partir du liquide à mesurer,
- à détecter la présence de liquide à un niveau haut prédéterminé (3,4) dans ledit récipient,
- à vider le récipient de mesure (1,2), caractérisé en ce qu'il consiste en outre à réduire la vitesse de remplissage dudit récipient de mesure (1,2) à l'approche dudit niveau prédéterminé.

9. Procédé de mesure selon la revendication 8, caractérisé en ce qu'il consiste à recueillir tout d'abord ledit liquide extrait du circuit de liquide de dialyse dans un récipient tampon (12), puis à remplir ledit récipient de mesure (1,2) à partir dudit récipient tampon (12).

10. Procédé de mesure selon l'une des revendications 8 et 9, caractérisé en ce qu'il consiste en outre à dégazer le liquide à mesurer avant de remplir ledit récipient de mesure (1,2).

11. Procédé de mesure selon l'une quelconque des revendications 8 à 10 caractérisé en ce qu'il consiste à remplir par gravité et à vider alternativement un premier (1) et un second (2) récipients de mesure à partir du liquide à mesurer présent dans ledit réservoir tampon (12).

## Patentansprüche

1. Gerät zur Messung der Menge der Flüssigkeit in einer künstlichen Niere, die vom Flüssigkeitskreislauf der Dialyse (10) abgezogen wurde und die der Ultrafiltration des Blutes entspricht, enthaltend:
- mindestens einen Meßbehälter (1,2) zur Aufnahme der abgezogenen Flüssigkeit, wobei dieser Behälter (1,2) mit mindestens einem Detektor für das hohe Niveau (3,4) ausgestattet ist, der in der Lage ist, die Anwesenheit der Flüssigkeit in einem vorherbestimmten Niveau nachzuweisen,
- Entleerungsmittel (6,7,8) dieses Meßbehälters,
dadurch gekennzeichnet, daß es ausserdem aufweist:
- einen Zwischenbehälter (12), der einerseits mit dem Flüssigkeitskreislauf der Dialyse (10) und andererseits mit dem meßbehälter (1,2) verbunden ist, um seine Füllung zu gewährleisten, wobei dieser Zwischenbehälter so angeordnet ist, daß die Füllung des Meßbehälters (1,2) durch Schwerkraft möglich ist.

2. Gerät nach Anspruch 1, dadurch gekennzeichnet, daß es einen ersten (1) und einen zweiten (2) Meßbehälter aufweist, die abwechselnd von dem Zwischenbehälter (12) gefüllt werden.

3. Gerät nach einem der Ansprüche 1 und 2, dadurch gekennzeichnet, daß die genannten Behälter (1,2,12) mit dem Atmosphärendruck verbunden sind.

4. Gerät nach einem der vorhergehenden Ansprüche, dadurch gekennzeichnet, daß die Meßbehälter (1,2) mindestens einen Teil aufweisen, wo der Querschnitt kleiner ist als der mittlere querschnitt der genannten Behälter und die genannten Detektoren für das Niveau (3,9,16) auf diesem Teil mit kleinerem Querschnitt angeordnet sind.

5. Gerät nach einem der vorhergehenden Ansprüche, dadurch gekennzeichnet, daß der Zwischenbehälter (12) mindestens einen Teil aufweist, der am Niveau der Detektoren (3,4,16) angeordnet ist und dessen Querschnitt größer oder gleich dem Querschnitt der Maßbehälter (1,2) bei diesem Niveau ist.

6. Gerät nach einem der vorhergehenden Ansprüche, dadurch gekennzeichnet, daß der Zwischenbahälter (12) an seinem oberen Teil mit dem genannten Flüssigkeitskreislauf der Dialyse (10) verbunden ist.

7. Gerät nach einem der vorhergehenden Ansprüche, dadurch gekennzeichnet, daß die genannten Behälter (1,2,12) durch ihren unteren Teil miteinander in Verbindung stehen.

8. Verfahren zur Messung der Menge der Flüssigkeit in einer künstlichen Niere, die vom Flüssigkeitskreislauf der Dialyse (10) abgezogen wurde und die der Ultrafiltration des Blutes entspricht, bestehend aus:
- Füllung mindestens eines Meßbehälters (1,2) mit der zu messenden Flüssigkeit,
- Nachweis der Anwesenheit einer Flüssigkeit an einem vorherbestimmten hohen Niveau (3,4) in diesem Behälter,
- Entleerung des meßbehälters (1,2),
dadurch gekennzeichnet, daß es ausserdem darin besteht, daß die Schnelligkeit der Auffüllung des genannten Meßbehälters (1,2) bei Annäherung an das genannte vorherbestimmte Niveau vermindert wird.

9. Meßverfahren nach Anspruch 8, dadurch gekennzeichnet, daß zuerst die genannte Flüssigkelt, die vom Flüssigkeitskreislauf der Dialyse abgezogen wurde, in einem Zwischenbehälter (12) gesammelt und dann der genannte Meßbehälter (1,2) vom Zwischenbehälter (12) gefüllt wird.

10. Meßverfahren nach einem der Ansprüche 8 und 9, dadurch gekennzeichnet, daß ausserdem die zu messende Flüssigkeit entgast wird, bevor der Meßbehälter (1,2) gefüllt wird.

11. Meßverfahren nach einem der Ansprüche 8 bis 10, dadurch gekennzeichnet, daß durch Schwerkraft abwechselnd der erste (1) und der zweite (2) der Meßbehälter gefüllt und entleert wird, ausgehend von der zu messenden Flüssigkeit im Zwischenbehälter (12).

## Claims

1. Device for measuring, in an artificial kidney, the quantity of liquid extracted from the dialysis liquid circuit (10) and corresponding to the ultrafiltration of the blood, comprising:
- at least one measuring container (1, 2) intended to receive the extracted liquid, the said container (1, 2) being provided with at least one high-level detector (3, 4) capable of detecting the presence of liquid at a predetermined level,
- means (6, 7, 8) for emptying the said measuring container characterised in that it also comprises:
- a buffer container (12) connected on the one hand to the dialysis liquid circuit (10) and connected on the other hand to the measuring container (1, 2) in order to provide for its filling, this buffer container being positioned so as to allow the gravity filling of the measuring container (1, 2).

2. Device according to Claim 1, characterised in that it comprises a first measuring container (1) and a second measuring container (2) which are alternately filled from the buffer container (12).

3. Device according to one of Claims 1 and 2, characterised in that the said containers (1, 2, 12) are connected to atmospheric pressure.

4. Device according to one of the preceding claims, characterised in that the measuring containers (1, 2) are provided with at least one part whose cross-section is smaller than the mean cross-section of the said containers, the said level-detectors (3, 4, 16) being situated on this part of smaller cross-section.

5. Device according to one of the preceding claims, characterised in that the buffer container (12) has at least one portion situated at the level of the said detectors (3, 4, 16) whose cross-section is greater than or equal to the cross-section of the measuring containers (1, 2) at the said level.

6. Device according to one of the preceding claims, characterised in that the said buffer container (12) is connected at its upper part to the said dialysis liquid circuit (10).

7. Device according to one of the preceding claims, characterised in that the said containers (1, 2, 12) are brought into communication via their lower part.

8. Method for measuring, in an artificial kidney, the quantity of liquid extracted from the dialysis liquid circuit (10) and corresponding to the ultrafiltration of the blood, consisting in:
- filling at least one measuring container (1, 2) with the liquid to be measured,
- detecting the presence of liquid at a predetermined high level (3, 4) in the said container,
- emptying the measuring container (1, 2),
characterised in that it also consists in reducing the rate of filling of the said measuring container (1, 2) as the said predetermined level is approached.

9. Measuring method according to Claim 8, characterised in that it consists in first collecting the said liquid extracted from the dialysis liquid circuit in a buffer container (12), then in filling the said measuring container (1, 2) from the said buffer container (12).

10. Measuring method according to one of Claims 8 and 9, characterised in that it also consists in degassing the liquid to be measured before filling the said measuring container (1, 2).

11. Measuring method according to any one of Claims 8 to 10, characterized in that it consists in alternately gravity filling and emptying a first measuring container (1) and a second measuring container (2) with the liquid to be measured present in the said buffer container (12).
